# EUROPEAN PATENT APPLICATION

(11) **EP 1 452 096 A1**
(43) Date of publication of application: **01.09.2004**
(21) Application number: 02783584.2
(22) Date of filing: 21.11.2002
(51) Int. Cl.: A23K 1/16

(54) **METHOD OF FATTENING POULTRY**

(30) Priority: 29.11.2001 JP 2001364356
(71) Applicant: Ajinomoto Co., Inc., Tokyo 104-0031 (JP)
(72) Inventor: BANNAI, Makoto c/o AJINOMOTO CO., INC., Kawasaki-Shi, Kanagawa 210-0801 (JP); SEKI, Shinobu c/o AJINOMOTO CO., INC., Kawasaki-Shi, Kanagawa 210-0801 (JP); TAKAHASHI, Michio c/o AJINOMOTO CO., INC., Kawasaki-Shi, Kanagawa 210-0801 (JP)
(74) Representative: HOFFMANN - EITLE
(86) International application number: PCT/JP2002/012163
(87) International publication number: WO 2003/045162

(57) **Abstract**

A method of fattening fowls comprising the step of administrating one or more running neuron inhibitory substance(s) is provided. Especially, a method of fattening fowls is provided, wherein fowls are administrated by a substance selected from the group consisting of GABA_{B} receptor agonist, GABA_{A} receptor agonist, GABA_{A} receptor enhancer, kainate receptor antagonist and a combination thereof or fowls are provide with a feed comprising the substance or a combination thereof.

## Description

### Background of the Invention

The present invention is directed to a method of fattening fowls and to a method of increasing a feed efficiency for fowls.

To fattening fowls, methods have been taken in which their motility was reduced by physically restricting their liberty such as confining them in a narrow space in combination with compulsive ingestion of a large amount of feed. Even in such cases, however, it has been generally known that fowls may consume the overtaken energy not by intentional behaviors but by non-intentional behaviors such as twitchily shaking themselves or by jiggling their heads, which would not lead to the expected results.

On the other hand, independent of the development of such methods of fattening fowls, many investigations have been focused on the running activity, especially on the night running activity, during the studies of the central nervous systems responsible for motility. For example, the inventors of the present invention reported that when the ventromedial nucleus of hypothalamus (hereinafter it may be referred to as "VMH") of rat was stimulated by water absorbent polymers, the running activity of rat was induced by the pressure stimulus (Yokawa, et. al., Physiology & behavior (1989), 46, 713-717). In this report, signals from VMH were indicated to be required for the induction of running activity in rat, based on the observation that the running activity did not occur when VMH region had been excised from the animals. Additionally, the inventors demonstrated that the foregoing induction of rat running activity caused by polymers could be inhibited by administration of GABA (γ-aminobutyric acid)(Yokawa, et al., Physiol. & Beav. (1990), 47, 1261-1264).

The inventors also reported that the running activity in rat was induced by kainate receptor agonists (Narita et al., Brain Res. (1993), 603, 243-247). According to this report, the running activity in rat was induced by kainate and was not inhibited by GABA, but was inhibited by DNQX (6,7-dinitroquinoxaline-2,3-dione), a kainate receptor antagonist, which suggests that neuron which is indirectly controlling the running activity in rat may be stimulated through kainate receptors and that GABA_{A} receptors presynaotically inhibit the neuron controlling the running activity in rat against the kainate receptors.

On the other hand, for GABA_{B} receptors, it has been also reported that substances having the competitive inhibitory activity against GABA_{B} receptors are likewise useful in treatment of diseases accompanied by convulsion, Alzheimer's disease or memory retention disorder (Japanese unexamined publication, JP 4-243853). However little has been known about the involvement of GABA_{A} receptors in motility.

### Summary of the Invention

The object of the present invention is to provide a method of fattening fowls (poultries).

The object of the present invention is also to provide a method of increasing feed efficiency for fowls.

The inventors have been suggested that there are some neurons in the VMH which were strongly suggested to be involved in the running activity in rat, especially in the night running activity, as previously mentioned. The inventors designated the neuron existing in the VMH region as "running neuron" and concluded that the running neuron is the neuron which regulates the non-intentional locomotion in rat. The inventors became to believe that the running neuron existed also in fowls and controlled non-intentional behaviors, from the point of view that behaviors, which could be recognized as non-intentional behaviors similarly as the night running activity in rat, such as twitchily shaking themselves or by jiggling their heads.

The inventors led to the present invention by considering that animals, especially fowls, can be fattened by inhibiting the non-intentional energy-consuming locomotion by inhibiting the running neuron, with the conception that animals generally ingest excessive energy and the behaviors such as running activity of rat in night or jiggling the head of fowls should be one of the "non-intentional behaviors" to non-intentionally consume the overtaken energy and are not the "intentional behaviors".

Thus, the present invention is a method of fattening fowls comprising administrating a substance which inhibits the running neuron to fowls.

In particular, the present invention is a method of fattening fowls comprising feeding fowls with a feed containing one or more substances which inhibit the running neuron.

More specifically, the present invention is a method of fattening fowls comprising administrating a substance selected from the group consisting of GABA_{B} receptor agonists, GABA_{A} receptor agonists, or substances that enhance the activity of GABA_{A} receptor, kainate receptor antagonists and any combination thereof or feeding fowls with a feed containing these substances or a combination thereof.

Additionally, the increase in body weight of the fowls per unit of a feed is improved by adding a running neuron inhibitory substance into the feed because the non-intentional behaviors of the fowls which ingested the feed would be inhibited and their energy consumption would be reduced. Therefore, the present invention is also a method of increasing feed efficiency comprising adding a substance which presynaptically or postsynaptically inhibit the running neuron.

### Brief Description of the Drawings

Fig. 1 is a graph showing the increase in body weight from day 21 to day 41 after birth when GABA was supplemented into the feed.
Fig. 2 is a graph showing the increase in body weight per 1 kg of feed from day 21 to day 41 after birth when GABA was supplemented into the feed. "*" indicates the experimental groups exhibiting a remarkable effects compared to the group where the addition rate of GABA was 0% (p < 0.05).

### Description of the Preferred Embodiments

The fowls which may be fattened by the present invention are those birds which may be reared, including, but not limited to, chicken, turkey, duck or goose.

The running neuron inhibitory substances which may be used for fattening fowls according to the present invention include GABA_{B} receptor agonists, GABA_{A} receptor agonists, GAGA_{A} receptor enhancers (substances which can affect GABA_{A} receptors and can enhance the activity the receptors) and kainate receptor antagonist.

Particularly, running neuron inhibitory substances may include, but are not limited to: GABA; isoguvacine (1,2,3,6- tetrahydro -4- pyrydinecarboxylic acid), musimol (5- aminomethyl -3- hydroxyisoxazole), THIP (4,5,6,7-tetrahydroisoxazolo [5,4-c] pyridine-3-ol) and the like as GABA_{A} receptor agonists; GABA, baclofen (4-amino-3-(4-chlorophenyl) butanoic acid), SKF97541(3- aminopropyl(methyl)phosphinic acid) and the like as GABA_{B} receptor agonists; benzodiazepine and the like as GABA_{A} receptor enhancers; and CNQX (6-cyano-7-nitroquinoxaline-2,3-dione), CNQX disodium salt (6-cyano-7-nitroquinoxaline-2,3-dione disodium), DNQX, GAMS (γ-D-glutamylamino methylsulphonic acid), NBQX (2,3-dioxo-6-nitro-1,2,3,4-tetrahydrobenzo[f]quinoxaline -7- sulphonamide), NBQX disodium (2,3- dioxo-6-nitro-1,2,3,4-tetrahydorbenzo[f]quinoxaline-7-sulphonamide disodium) and the like as kainate receptor antagonists.

To determining whether the given substance has the running neuron inhibitory activity, a bioassay system can be used. For example, a 26-gauge stainless guide cannula may be implanted in rat's VMH and the rats are allowed at least for seven days for the surgical recovery. After recovery period, about 100 pmol of kainic acid in about 0.5 µl saline is injected through the internal cannula to ascertain that the guide cannula position hits the running neuron. The rats that do not express running behavior, which means that they do not respond to the treatment, will be omitted from the following experiments. Three days later, the mixture of kainate (about 100 pmol) and the putative inhibitory compound (about 50 pmol to about 50 nmol) may be injected through the cannula. When the compound injected simultaneously with kainate either reduces or abolishes the kainic acid-induced running activity, that compound can be recognized as the running neuron inhibitory substance.

When the fowls are fattened according to the present invention, the running neuron inhibitory substances alone or together with appropriate additives may be administered orally or parenterally. In general, they are preferably administered orally.

When the running neuron inhibitory substances are orally administered according to the present invention, they may be administered as a preparation admixed with additional non-toxic substances such as sweetening, fragrances, coloring matters, preservatives or emulsifiers. Such additives include, for example, saccharides such as lactose and galactose, starches such as cornstarch, fatty acid salts such as magnesium stearate, alginic acid, talc and polyethylene glycol. According to the present invention, the running neuron inhibitory substances can be administered in the form of capsules, tablets, or syrups and the like, or may be administered by adding them into the feed or drinking water.

When the running neuron inhibitory substances are parenterally administered, they may be administered transcutaneously, intravenously, intraperitoneal, ophthalmologically, by inhalation or transnasally.

In any cases, the total dose, concentration and frequency will be determined depending to the age, general condition, body weight, fattening scheme, of the fowls or the desired effects and the like.

When the fowls are fattening according to the present invention, it is preferable to orally administer the running neuron inhibitory substances to the reared fowls by adding the substances into the ingesta or drinking water taken by the fowls, preferably by administrating the substances by adding them into the feed. When adding the running neuron inhibitory substances into the feed, they may be added during the manufacturing process of feeds or they may be added after the feeds are produced. The amount of the running neuron inhibitory substances in the feed will be determined depending on the age, general condition, body weight, fattening scheme, the intake, of the fowls or the desired effects and the like.

When the running neuron inhibitory substances are orally administered according to the present invention, the running neuron inhibitory substances are typically administered to the-fowls such that the given substances would be about 0.1g/kg body weight to about 3g/kg body weight, preferably about 0.25g/kg body weight to about 2g/kg body weight, more preferably about 0.5g/kg body weight to about 1g/kg body weight per day. For example, the running neuron inhibitory substances may be incorporated into the intakes preferably including feed at a rate of about 0.25 to about 3% by weight, more preferably about 5 to about 1.5% by weight. When a plurality of oral administration form are combined such as in the case where the running neuron inhibitory substances are incorporated into a plurality of the intakes, the total amount of the running neuron inhibitory substances will be adjusted such that the total amount would be within the aforementioned range.

The running neuron inhibitory substances or the intakes including the feeds containing thereof are generally given under the environment where the fowls can ingest them ad lib. However, The running neuron inhibitory substances or the feeds containing thereof and the like can be given only during the defined period or at a particular day or time. For example, they may be received only in the morning, or only during one to several days, or they may be continuously received. Particular, for fowls it is preferable to administer the substances at the growing period, for example from 3 weeks to 6 weeks after birth.

The feeds to which the running neuron inhibitory substances are incorporated at the aforementioned ratio are generally improved in the feed efficiency. Thus, it would be possible to reduce the required amount of the feed in "kg" per 1 kg of fowl body weight-increase by using such feeds, compared to the cases where the feed free from the running neuron inhibitory substances are used. Based on this aspect of the present invention, the present invention is a method of increasing the feed efficiency for fowls. In this context, the feed efficiency is experimentally calculated by the formula, (intake of the feed during the measuring period)/(increase of body weight during the measuring period), which is the indicator of the effects of feed consumption on the increase in body weight.

When the running neuron inhibitory substances are parenterally administered according to the method of the present invention, the administration includes transcutaneous administration, intravenous administration, intraperitoneal administration, ophthalmologic administration, inhalation or transnasal administration. For parenteral administration according to the present invention, the running neuron inhibitory substances may be generally contained at about 0.01 to 30% by weight, preferably 0.05 to 20% by weight based on the total weight of the preparation. When the running neuron inhibitory substances are parenterally administered according to the method of the present invention, the running neuron inhibitory substances may be formulated with additional physiologically acceptable carriers such as preservatives or osmoragulators and the like.

When the running neuron inhibitory substances are parenterally administered, the substances would be administered at about 10mg/kg body weight to about 300mg/kg body weight, preferably about 25mg/kg body weight to about 200mg/body weight, more preferably about 50mg/kg body weight to about 100mg/body weight per day. The dose frequency will be determined depending on the age, general condition, body weight, fattening scheme, of the fowls or the desired effects and the like. Generally, they may be administered one to about 6 times per day, preferably one to about 3 times a day.

When fowls are fattened according to the method of the present invention, the intake of the fowls may transiently increase in some cases, but after that the intake generally tends to decrease and to remain at the level of normal intake or at the level slightly higher than the normal level. When fowls are fattened according to the method of the present invention, the fowls can be bred under the conventional condition except that they received the running neuron inhibitory substance(s). Thus, the fowls that received the running neuron inhibitory substances can be kept in the similar rearing facility and environment. For example, it is not necessary to place the fowls in the environment where the intentional behaviours are more extensively inhibited than in the conventional cases, such as confining them within a narrower space. On the other hand, even if the fowls are reared in the large space where they can move without restraint, the purpose of the invention would be achieved because the energy consuming non-intentional behaviors would be inhibited according to the present invention. Thus, giving the running neuron inhibitory substances to fowls according to the invention, without combining it with additional means, can increase the body weight of fowls.

### Examples

### Example 1

120 Arbor Acres, a representative species of broilers were used for the experiments. Four (4) experimental groups were prepared, each of which was given a feed containing GABA at 0%, 0.5%, 1% or 3% by weight. Six gages for each group were used and 5 individuals were used for one gage. The gage was 1m x 1m, the floor was concrete which was covered by bran.

The feed was prepared based on the composition of Table 1 and Table 2 and was given until day 21 after birth. The fattening feed having the composition of Table 3 and Table 4 was given from day 21 to day 41 after birth. During the experimental period, the feed and water was freely provided. The feed was given as a mash feed. The vaccines against Newcastle disease and against infectious bronchitis were given to 7 days old and 14 days old broilers. The temperature was 26 - 33°C and the humidity was 75 - 85% during the experimental period. The data was subjected to variance analysis and then analyzed by Fisher's PLSD method as a post-hock test and determined as significant difference when p<0.05.

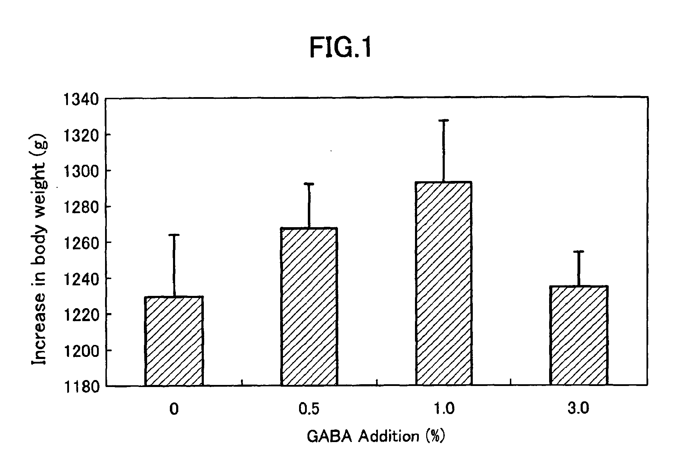

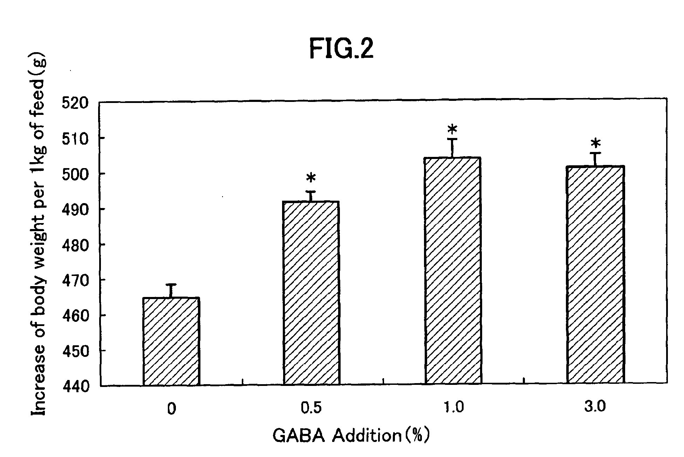

In Figure 1, the horizontal axis indicates the additive rate of GABA into the feed and the longitudinal axis indicates the increase in the body weight from day 21 to day 41 after birth. The maximum increase was observed at 1% additive rate of GABA. The tendency of increase was also observed at 0.5% but the significant difference was not clearly observed. The increase in the body weight per 1 kg of feed during day 21 to day 41 after birth is indicated in Figure 2. In the experimental group where the GABA additive rate was 0.5%, 1 % or 3%, a significant increase was shown in each group when compared to 0% group.

These results indicated that the tendency of increasing body weight was observed and the increasing rate of body weight per 1 kg of feed was significantly elevated.

**Table 1.**

| Composition of initial (until day 21 after birth) feed | |
|---|---|
| Ingredient | (%) |
| Corn (crude protein 8.38%) | 52.606 |
| inert expander (fine sand) | 3.000 |
| un-defatted soybean meal (crude protein 37.19%) | 15.000 |
| soybean meal (crude protein 46.81 %) | 18.466 |
| fish meal (crude protein 62.18%) | 5.000 |
| palm oil | 2.785 |
| DL-methionine | 0.174 |
| limestone (35.69%) | 1.385 |
| Ca(H₂PO₄) (calcium 15.03%, phosphate 20.06%) | 1.074 |
| salt | 0.260 |
| Vitamin/mineral premix | 0.250 |
| Total (%) | 100.000 |

**Table 2.**

| Nutrient composition of initial (until day 21 after birth) feed | |
|---|---|
| Nutrient composition | |
| dry matter | 88.79% |
| metabolic calories of fowls | 3050 Kcal/kg |
| crude protein | 21.50% |
| crude fat | 8.15% |
| crude fiber | 3.50% |
| lysine | 1.26% |
| arginine | 1.40% |
| methionine | 0.54% |
| methionine + cysteine | 0.90% |
| threonine | 0.83% |
| tryptophan | 0.26% |
| isoleucine | 0.82% |
| leucine | 1.84% |
| valine | 1.02% |
| calcium | 1.00% |
| total phosphate | 0.70% |
| phosphate excluding phytate | 0.45% |
| salt | 0.40% |

**Table 3.**

| Composition of feed for growing period (from day 21 to day 41 after birth) | | | | |
|---|---|---|---|---|
| Ingredient | Control group (%) | 0.5% GABA (%) | 1.0% GABA (%) | 3.0% GABA (%) |
| Corn (crude protein 8.38%) | 55.7 | 55.7 | 55.7 | 55.7 |
| inert expander (fine sand) | 3.00 | 2.50 | 2.00 | |
| GABA | | 0.500 | 1.00 | 3.00 |
| un-defatted soybean meal (crude protein 37.19%) | 15.0 | 15.0 | 15.0 | 15.0 |
| soybean meal (crude protein 46.81 %) | 14.9 | 14.9 | 14.9 | 14.9 |
| fish meal (crude protein. 18%) | 5.00 | 5.00 | 5.00 | 5.00 |
| palm oil | 3.79 | 3.79 | 3.79 | 3.79 |
| DL-methionine | 0.031 | 0.031 | 0.031 | 0.031 |
| limestone (35.69%) | 1.22 | 1.22 | 1.22 | 1.22 |
| Ca(H₂PO₄)₂ (calcium 15.03%, phosphate 20.06%) | 0.862 | 0.862 | 0.862 | 0.862 |
| salt | 0.263 | 0.263 | 0.263 | 0.263 |
| Vitamin/mineral premix | 0.250 | 0.250 | 0.250 | 0.250 |
| Total (%) | 100 | 100 | 100 | 100 |

**Table 4.**

| Nutrient composition of feed for growing period (from day 21 to day 41 after birth) | | | | |
|---|---|---|---|---|
| Nutrient composition | Control group | 0.5% xGABA | 1.0% GABA | 3.0% GABA |
| dry matter (%) | 88.8 | 88.8 | 88.8 | 88.8 |
| metabolic calories of fowls (Kcal/kg) | 3150 | 3150 | 3150 | 3150 |
| crude protein (%) | 20.0 | 20.0 | 20.0 | 20.0 |
| crude fat (%) | 9.23 | 9.23 | 9.23 | 9.23 |
| crude fiber (%) | 3.33 | 3.33 | 3.33 | 3.33 |
| lysine (%) | 1.16 | 1.16 | 1.16 | 1.16 |
| arginine (%) | 1.29 | 1.29 | 1.29 | 1.29 |
| methionine (%) | 0.38 | 0.38 | 0.38 | 0.38 |
| methionine + cysteine (%) | 0.72 | 0.72 | 0.72 | 0.72 |
| threonine (%) | 0.78 | 0.78 | 0.78 | 0.78 |
| tryptophan (%) | 0.24 | 0.24 | 0.24 | 0.24 |
| isoleucine (%) | 0.75 | 0.75 | 0.75 | 0.75 |
| leucine (%) | 1.74 | 1.74 | 1.74 | 1.74 |
| valine (%) | 0.95 | 0.95 | 0.95 | 0.95 |
| calcium (%) | 0.90 | 0.90 | 0.90 | 0.90 |
| total phosphate (%) | 0.64 | 0.64 | 0.64 | 0.64 |
| phosphate excluding phytate (%) | 0.40 | 0.40 | 0.40 | 0.40 |
| salt (%) | 0.40 | 0.40 | 0.40 | 0.40 |

According to the present invention, it is possible to fatten fowls. Additionally, the feed efficiency can be increased and the fowls can be efficiently fattened according to the present invention. In particular, the present invention allow to fatten the fowls, without using additional other means and without imposing a burden on the fowls.

## Claims

1. A method of fattening fowls, which comprises administrating one or more running neuron inhibitory substance(s) to the fowls.

2. The method according to claim 1, which comprises giving a feed containing one or more running neuron inhibitory substance(s).

3. The method according to claim 1 or 2, wherein the running neuron substances are selected from the group consisting of GABA_{B} receptor agonist, GABA_{A} receptor agonist, GABA_{A} receptor enhancer, kainate receptor antagonist and a combination thereof.

4. The method according to claim 3, wherein the running neuron inhibitory substance is GABA_{A} receptor agonist.

5. The method according to claim 3, wherein the running neuron inhibitory substance is GABA_{B} receptor agonist.

6. The method according to claim 4, wherein GABA_{A} receptor agonist is selected from the group consisting of GABA, isoguvacine, muscimol and THIP.

7. The method according to claim 5, wherein GABA_{B} receptor agonist is selected from the group consisting of GABA, baclofen and SKF97541.

8. The method according to claim 3, wherein GABA_{A} receptor enhancer is benzodiazepine.

9. The method according to claim 3, wherein kainate receptor antagonist is selected from the group consisting of CNQX, CNQX disodium salt, DNQX, GAMS, NBQX, NBQX disodium.

10. The method according to any one of claims 1 to 9, wherein the running neuron inhibitory substance is administrated during the period of 3 weeks to 6 weeks after birth.
